# EUROPEAN PATENT SPECIFICATION

(11) **EP 1 275 339 B1**
(45) Date of publication and mention of the grant of the patent: **21.09.2005**
(21) Application number: 02014845.8
(22) Date of filing: 03.07.2002
(51) Int. Cl.: A61B 5/00

(54) **Risk check sheet for dental caries**
Gefahrenüberprüfungsblatt für Zahnkaries
Feuille de contrôle des risques de caries dentaires

(30) Priority: 10.07.2001 JP 2001209606
(43) Date of publication of application: 15.01.2003
(73) Proprietor: GC Corporation, Tokyo 174 (JP)
(72) Inventor: Tosaki, Satoshi, GC Corporation, Tokyo 174 (JP); Nishikata, Hiromi, GC Corporation, Tokyo 174 (JP); Kumaoka, Masayuki, GC Corporation, Tokyo 174 (JP); Watanabe, Yoshiko, GC Corporation, Tokyo 174 (JP)
(74) Representative: Müller-Boré & Partner Patentanwälte

(56) References cited:
- EP-A- 0 877 249
- WO-A-00/74559
- WO-A-97/50046

## Description

### BACKGROUND OF THE INVENTION

### 1. Field of the Invention:

The present invention relates to a risk check sheet for dental caries, which enables a technician for dental care to grasp the risk of dental caries of a patient and to show its state to the patient in an easily understandable way.

### 2. Description of the Conventional Art:

Document WO-A-0 074 559 discloses a risk check sheet according to the preample of claim 1.

In order to consider a measure for inhibiting the progress of "dental caries" as a condition-pluricausal disease caused by the intra-oral state or life habit and to plan for treating it, a movement in which a technician for dental care confirms the intra-oral state and life habit of a patient through inquiries or inspections and grasps "whether easily or hardly to be attacked by the dental caries", that is, "a risk of dental caries", of the patient per se starts to occur. To make the patient per se understand the risk of dental caries is necessary in giving a motivation against the maintenance and continuation of oral care.

For example, the caries risk radar chart as disclosed in *Clinical Cariology* (published by Ishiyaku Publishers, Inc.) is concerned with a method in which among the results obtained by checking the amount of dental plaque, the level of *Mutans streptococci,* the level of Lactobacilli, the number of eating and drinking per day, the amount and quality of saliva, the buffer capacity of saliva, DMFT (an index showing the caries experience of permanent teeth, which is expressed by a value obtained by dividing the sum of caries-experienced teeth of permanent teeth of a subject by the number of subjects), the shape of fissure in enamel, the fluoride mouth wash, the fluoride application, the family make-up, the level of bacteria of the family, and the like by using a "Caries Risk Questionnaire", the eight major factors, i.e., the buffer capacity of saliva, the level of *Mutans stroptococci,* the level of Lactobacilli, the number of eating and drinking, the accumulated amount of dental plaque, the use status of fluoride, the DMFT, and the amount and quality of saliva, are selected as parameters related to the risk of dental caries and plotted in the radar chart, and the risk of dental caries is confirmed from the size and shape of a graph thus drawn. Accordingly, the risk of dental caries can be simply shown in a visual form. However, not only the number of the parameters related to the risk of dental caries is high and the procedures are complicated, but also each of the parameters uses technical terminologies, which is difficult for a general patient to understand. Therefore, it has been difficult to make the patient per se understand the risk of dental caries and give a motivation for the maintenance and continuation of oral care.

### SUMMARY OF THE INVENTION

The invention is aimed to overcome the above-described problems of the conventional art and to provide a risk check sheet for dental caries, which enables a technician for dental care to grasp the risk of dental caries of a patient by confirming the intra-oral state and life habit, particularly the eating habit, of the patient from the diagnosis results obtained through inquiries or inspections or by utilizing instruments, to comprehensively show its state to the patient in an easily understandable way, and to give the patient a motivation for the maintenance and continuation of oral care.

We, the present inventors made extensive and intensive investigations to achieve the above-described aim. As a result, they paid an attention to the point that, taking into account the matter that a dental caries is generated only when all of three factors, i.e., the quality of teeth and saliva of a patient, the intra-oral bacterium (dental plaque) and the foods, are under certain conditions (see Keyes PH: Recent advances in dental caries research, bacteriology, bacteriological findings, and biological implications, *Int Dent J*, 12, 443 (1962)), and considering the above reversely from the standpoint on how to inhibit the dental caries, if any of the foregoing conditions could be improved, the dental caries would not be generated. Finally, it has been found that when the parameters considered hitherto to relate to the risk for the generation of dental caries, been as disclosed in the above-cited *"Clinical Cariology"*, are summarized into the above-described three factors and numerical together, and each of the numerical risks of dental caries is displayed in a specific method, a technician for dental care can make a patient comprehensively understand the risk of dental caries and give the patient a motivation for the maintenance and continuation of oral care, leading to accomplishment of the invention.

Specifically, the invention relates to a risk check sheet for dental caries that is used for judging the risk of dental caries, which comprises a list sheet provided with three kinds of risk items consisting of a risk of dental caries caused by a resistance, a risk of dental caries caused by intra-oral bacteria, and a risk of dental caries caused by eating habit, each having a plurality of check items, as to each of which one from a plurality of selection items having different ranks is to be alternatively selected, and numerical data of the risks of dental caries by summing up evaluation scores given in the selection items for each risk item; and a chart sheet provided with a standard circle having a predetermined radius and centering around an origin, standard lines consisting of three lines radially provided with a distance of 120 degree, centering around the origin, each starting at least from the standard circle, and allocated for the three kinds of risks of dental caries, and indicator circles, each of which is drawn such that, when a point of intersection between each standard line and the standard circle is defined as a front point of intersection, whereas a position symmetrical to the front point of intersection on the standard circle is defined as a rear point of intersection, a length between each of points obtained by dividing equally a length from the front point of intersection to a maximum point positioned in a predetermined distance longer than the radius of the standard circle on the standard line by a maximum value of the sum of evaluation scores in each corresponding risk item and the rear point of intersection is a diameter.

Thus, in the risk check sheet for dental caries having such a configuration, it has also been found to be preferred that the check item with respect to the risk of dental caries caused by a resistance is constituted of at least two kinds selected from the group consisting of the number of decayed teeth at present, the number of filled teeth in the past, the number of missing teeth, the status of a set of teeth, the status of gingiva, the amount of saliva, the buffer capacity of saliva, the age, the presence and kind of systemic disease, the presence of pregnancy, and the presence and kind of a drug administered; the check item with respect to the risk of dental caries caused by intra-oral bacteria is constituted of at least two kinds selected from the group consisting of the amount of dental plaque, the level of *Mutans streptococci,* the level of *Sorbrinus streptococci,* and the level of Lactobacilli; and the check item with respect to the risk of dental caries caused by eating habit is constituted of at least two kinds selected from the group consisting of the number of eating and drinking per day, the frequency of eating between meals per day, and the contents of eating between meals.

### BRIEF DESCRIPTION OF THE DRAWINGS

Fig. 1 is an explanatory view showing one embodiment of a list sheet of a risk check sheet for dental caries according to the present invention.
Fig. 2 is an explanatory view showing one embodiment of a chart sheet of a risk check sheet for dental caries according to the present invention.
Fig. 3 is an explanatory view showing one example in which an entry is made in the list sheet as shown in Fig. 1.
Fig. 4 is an explanatory view showing one example in which the results of the list sheet as shown in Fig. 1 are entered in the chart sheet as shown in Fig. 2.

### DESCPRION OF THE PREFERRED EMBODIMENTS

The risk check sheet for dental caries according to the present invention will be hereunder described in detail with reference to the accompanying drawings.

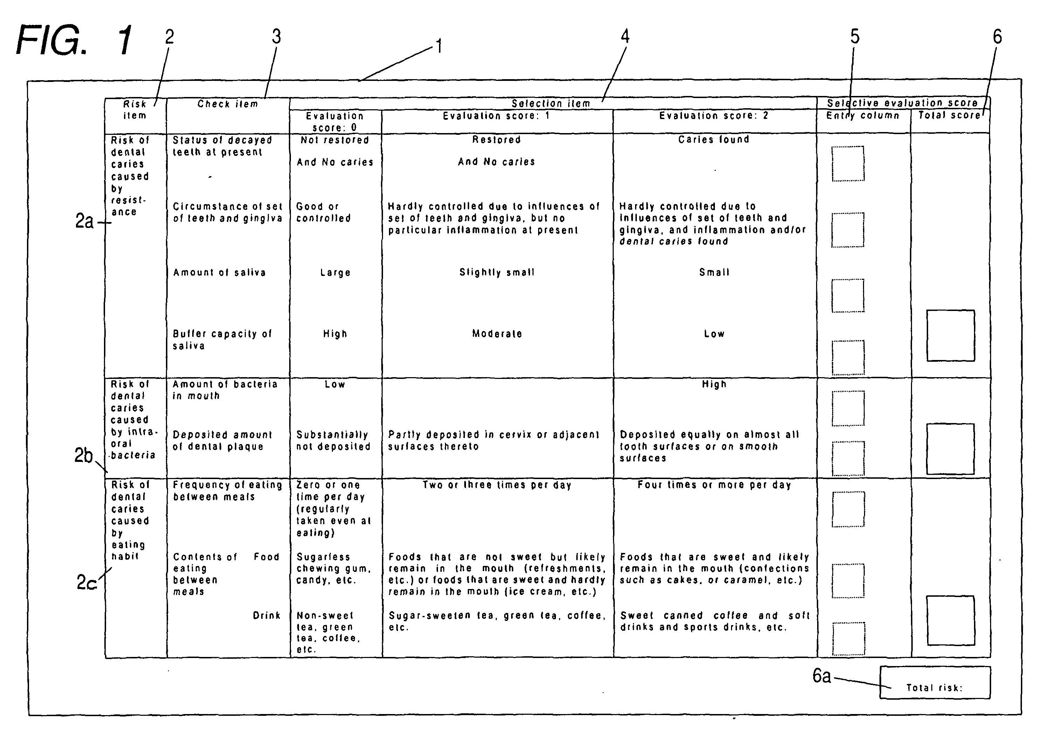

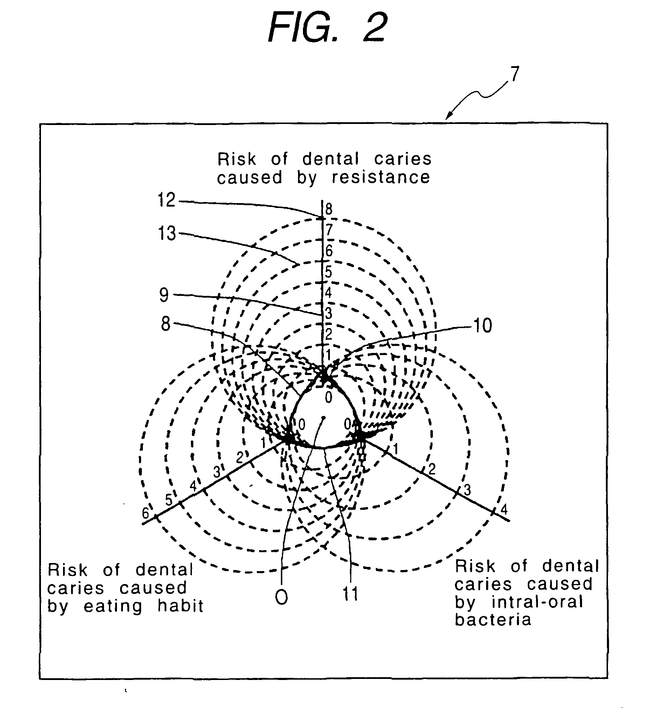

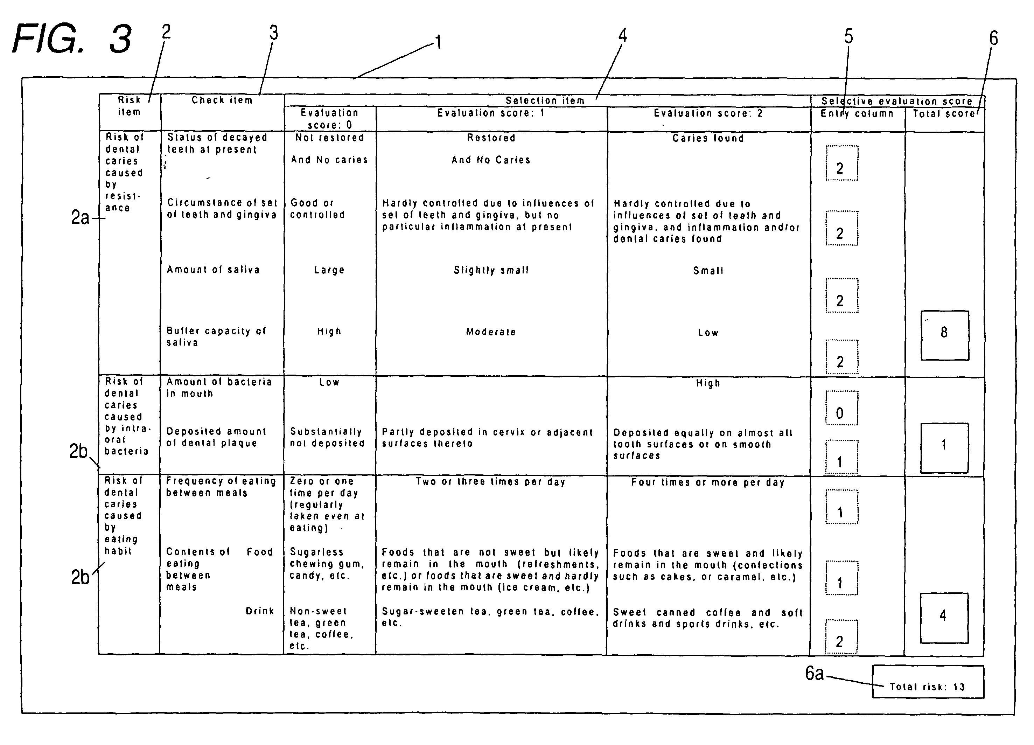

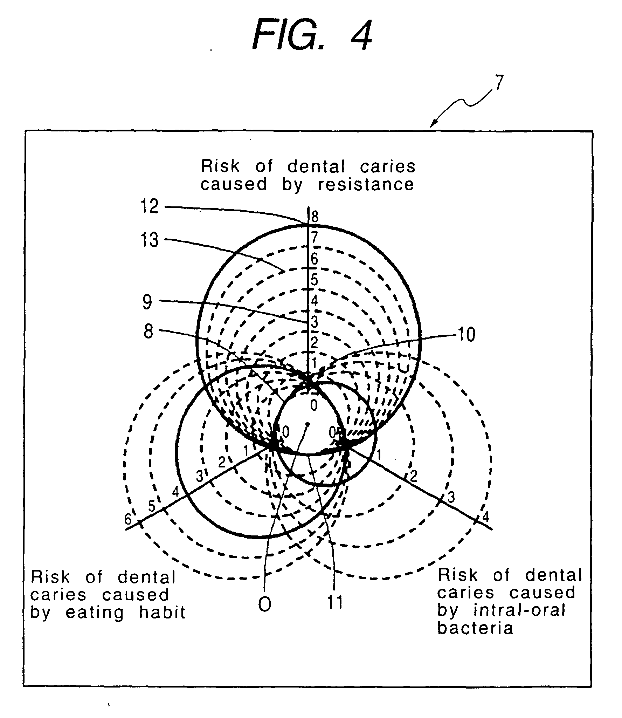

Fig. 1 is an explanatory view showing one embodiment of a list sheet of a risk check sheet for dental caries according to the present invention; Fig. 2 is an explanatory view showing one embodiment of a chart sheet of a risk check sheet for dental caries according to the present invention; Fig. 3 is an explanatory view showing one example in which an entry is made in the list sheet as shown in Fig. 1; and Fig. 4 is an explanatory view showing one example in which the results of the list sheet as shown in Fig. 1 are entered in the chart sheet as shown in Fig. 2.

In the drawings, a numeral 1 is a list sheet configuring a one side sheet of the risk check sheet for dental caries according to the present invention, which is used for numerical the risks of dental caries of a subject. This list sheet 1 expresses three kinds of risk items 2, as constituted of a risk 2a of dental caries caused by a resistance, a risk 2b of dental caries caused by intra-oral bacteria, and a risk 2c of dental caries caused by eating habit on a basis of the three factors proposed by Keyes as cited above. Each of these three kinds of the risk items 2 has a plurality of check items 3, as to each of which one from a plurality of selection items 4 having different ranks is alternatively selected, the selection items being ranked and given with an evaluation scores depending on the easiness to cause a dental caries. Thus, it is possible to numerical data of the risk of dental caries by summing up the evaluation scores given in the selection items 4 for each risk item 2.

In the risk 2a of dental caries caused by a resistance, expressed is a check item 3 related to the easiness to cause a dental caries by a physical strength and immunity of a subject. It is preferred that the check item 3 with respect to the risk 2a of dental caries caused by a resistance is constituted of at least two kinds selected from the group consisting of the number of decayed teeth at present, the number of filled teeth in the past, the number of missing teeth, the status of a set of teeth, the status of gingiva, the amount of saliva, the buffer capacity of saliva, the age, the presence and kind of systemic disease, the presence of pregnancy, and the presence and kind of a drug administered. In the embodiment shown in Fig. 1, selected are four items consisting of the status of decayed teeth at present, the circumstance of a set of teeth and gingiva, the amount of saliva, and the buffer capacity of saliva.

In the risk 2b of dental caries caused by intra-oral bacteria, expressed is a check item 3 related to the easiness to cause a dental caries by the state of intra-oral bacteria of a subject. It is preferred that the check item 3 with respect to the risk 2b of dental caries caused by intra-oral bacteria is constituted of at least two kinds selected from the group consisting of the amount of dental plaque, the level of *Mutans streptococci,* the level of *Sorbrinus streptococci,* and the level of Lactobacilli. In the embodiment shown in Fig. 1, selected are two items consisting of the amount (level) of bacteria in a mouth (not particularly limiting the kind of bacteria) and the deposited amount of a dental plaque.

In the risk 2c of dental caries caused by eating habit, expressed is a check item 3 related to the easiness to cause dental caries by the eating habit of a subject. It is preferred that the check item 3 with respect to the risk 2c of dental caries caused by eating habit is constituted of at least two kinds selected from the group consisting of the number of eating and drinking per day, the frequency of eating between meals per day, and the contents of eating between meals. In the embodiment shown in Fig. 1, selected are three items consisting of the frequency of eating between meals and the contents of eating between meals (foods and drinks).

The expressions used in the risk item 2 and the check item 3 of the list sheet 1 and those used in a chart sheet 7 as described later are not limited to those described above. For example, as a matter of course, the expression "number of decayed teeth at present" may be replaced by one having clearly the same meanings such as "number of currently decayed teeth". Also, it is preferred that every risk item is classified by coloring.

Preferably, the list sheet 1 is provided with an entry column 5 of selective evaluation score for entering an evaluation score given in the selection item 4 alternatively selected from the selection items 4 as to each check item 3 for each risk item 2 as well as with an entry column 6 of total score of the selective evaluation score. In the embodiment shown in Fig. 1, a natural number is employed as the selective evaluation score. As a method for obtaining such an evaluation score, for example, when the "number of decayed teeth at present" is concerned, there are a method in which the number of decayed teeth at present is answered directly in terms of a numerical value and a method in which ranges such as "0", "1 to 3", "4 to 7", etc. are prepared in advance. When the number of decayed teeth at present based on the answer is, for example, in the range of 1 to 3, the evaluation score may be entered as "1". As a method for entering the answer to each of the selection items 4, there are a method in which a numerical value is entered directly in the entry column 5 of selective evaluation score, as shown in Fig. 1 and a method in which circles are given on lines into which numerical values are entered as seen in usual questionnaires or check sheets, etc. Further, it is convenient to provide an entry column 6a of total risk such that the evaluation scores given in all of the risk items 2 are expressed together, whereby the total risk of dental caries can be confirmed.

When the answer to each of the selection items 4 is obtained, the answers are summed up for each risk item 2 based on the evaluation score given in each selection item 4 to make data. The method for summing up the answers is made based on the total score of the respective evaluation scores depending on the contents of the check items 3 for each risk item 2. Accordingly, it is convenient that the entry column 6 of total score of the selective evaluation scores is provided to display the data having been summed up. Further, in order to give an importance to the respective evaluation score depending on the contents of the risk item 2 or the check item 3, an arbitrary coefficient may be multiplied. Moreover, in order to entry the data in the chart sheet 7 as described later, it is preferred that the ultimate evaluation score of each risk item 2 is a natural number from which a fraction has been treated. In the embodiment shown in Fig. 1, since the evaluation score in each of the check items 3 is designated as 0, 1 and 2, respectively, no fraction is formed in the total score of the selective evaluation scores.

For example, as in an example shown in Fig. 3, the risk sheet 1 is entered in the following manner. That is, in the entry column of selective evaluation score of each of the selection items of the risk 2a caused by a resistance, entered are the evaluation scores "2", "2", "2" and "2", and in the entry column 6 of total score, entered is "8"; in the entry column of selective evaluation score of each of the selection items of the risk 2b caused by intra-oral bacteria, entered are the evaluation scores "0" and "1", and in the entry column 6 of total score, entered is "1"; in the entry column of selective evaluation score of each of the selection items of the risk 2c caused by eating habit, entered are the evaluation scores "1", "1" and "2", and in the entry column 6 of total score, entered is "4"; and in the entry column 6a of total risk, entered is "13" (i.e., 8 + 1 + 4).

Thus, a circle corresponding to the total evaluation score obtained from the evaluation scores having been summed up for each risk item 2 as numerical by the risk sheet 1 is drawn on the chart sheet 7. From the size of this circle, the relation among the respective risk items 2 is grasped, thereby making the risk of dental caries clear. Next, the chart sheet 7 will be described.

The chart sheet 7 is provided with a standard circle 8 having a predetermined radius and centering around an origin 0; standard lines 9 consisting of three lines radially provided with a distance of 120 degree, centering around the origin 0, each starting at least from the standard circle 8, and allocated for the three kinds of risks of dental caries consisting of the risk 2a of dental caries caused by a resistance, the risk 2b of dental caries caused by intra-oral bacteria, and the risk 2c of dental caries caused by eating habit; and indicator circles 13, each of which is drawn such that, when a point of intersection between each standard line 9 and the standard circle 8 is defined as a front point 10 of intersection, whereas a position symmetrical to the front point 10 of intersection on the standard circle 8 is defined as a rear point 11 of intersection, a length between each of points obtained by dividing equally a length from the front point 10 of intersection to a maximum point 12 positioned in a predetermined distance longer than the radius of the standard circle 8 on the standard line 9 by a maximum value of the sum of evaluation scores in each corresponding risk item 2 and the rear point 11 of intersection is a diameter.

In the embodiment shown in Fig. 2, the scales of the three kinds of risks of dental caries as described above, i.e., the risk 2a of dental caries caused by a resistance, the risk 2b of dental caries caused by intra-oral bacteria, and the risk 2c of dental caries caused by eating habit, are "8", "4" and "6", respectively in terms of the maximum sum of evaluation scores. Accordingly, there are provided the indicator circles 13, each of which is drawn such that a length between each of points obtained by dividing equally a length from the front point 10 of intersection, which is a point of intersection between each standard line 9 and the standard circle 8, to the maximum point 12 by each maximum value (i.e., "8", "4" and "6") of the sum of evaluation scores in each corresponding risk item 2 and the rear point 11 of intersection is a diameter.

Preferably, the indicator circle 13 is drawn by a tint line as compared with a usual line and/or a broken line, as in the embodiment shown in Fig. 2. Since the standard circle 8 expresses a minimum value of the data, the standard circle 8 is preferably drawn by a solid line with a usual shade. While it is preferred that each of the standard lines 9 starts from the standard circle 8, and no line is present within the standard circle 8, each of the standard lines 9 may start from the origin 0 within the standard circle 8. Further, it is preferred that in the indicator circles 13, its outer peripheral lines or a plane surrounded by a maximum circle thereof is displayed with a different color corresponding to each of the risk items 2.

On the chart sheet 7, a circle corresponding to the total evaluation score obtained from the evaluation scores having been summed up for each risk item 2 as numerical by the risk sheet 1 is drawn along the indicator circle 13. When a circle corresponding to the total evaluation score obtained from the evaluation scores having been summed up for each risk item 2 is drawn along the indicator circle 13 in such a way, a degree of the risk of dental caries in each of the risk items 2 becomes definite from the size of the circle. Further, it is possible to grasp the relation among the risks of dental caries from the size of a portion where the three circles overlap.

In the case of the example shown in Fig. 3, as in an example shown in Fig. 4, the chart sheet 7 is entered in the following manner. That is, since the total evaluation score of the risk 2a of dental caries caused by a resistance, the total evaluation score of the risk 2b caused by intra-oral bacteria, and the total evaluation score of the risk 2c caused by eating habit are "8", "1", and "4", respectively, circles corresponding to the respective evaluation scores of "8", "1", and "4" are entered along the indicator circles 13. In this situation, since the risk 2a of dental caries caused by a resistance is the largest, a technician for dental care will give a patient an advice such as the necessity for treating the dental caries immediately; and since the risk 2c of dental caries caused by eating habit is next large, a technician for dental care will give a patient an advice such that the intake of sweet drinks must be reduced.

In the risk check sheet for dental caries according to the present invention, even a patient who has been judged to have a high risk of dental caries can be largely suppressed with respect to the risk of dental caries. In contrast, even for a patient who has been judged to have a low risk of dental caries, when the patient is careless, the risk of dental caries will possibly increase. Accordingly, in addition to the above-described three kinds of risks of dental caries, that is, the risk 2a of dental caries caused by a resistance, the risk 2b of dental caries caused by intra-oral bacteria, and the risk 2c of dental caries caused by eating habit, a check item regarding the self management or the prophylactic factors of dental caries may be provided. Preferably, the check item regarding the prophylactic factors of dental caries is constituted of at least two kinds selected from the group consisting of the frequency of tooth brushing, the use of a dental floss, the use of an interdental brush, the use of a fluoride-incorporated tooth paste, the experience of local application of a fluoride solution, the experience of professional tooth cleaning, the experience of filling of pit and fissure, the frequency of going to dental clinic, and the habit of chewing gum. Each of the check items is provided with a plurality of selection items having different ranks to be alternatively selected, and data of the prophylactic factor of dental caries from the evaluation scores given in the selection items are summed up and numericalized. For example, when the resulting value is added to the value of the total risk, or if the case may be, is reduced therefrom, it is possible to comprehensively grasp the risk of dental caries and the prophylactic factor of dental caries.

When the check item regarding the prophylactic factor of dental caries is provided in such a way to check the prophylactic factor of dental caries caused by the self management or the prophylactic treatment, a technician for dental care can effectively use the risk check sheet for dental caries in planning for the treatment or giving an advice, and hence, such is preferred.

Incidentally, the risk sheet 1 and the chart sheet 7 constituting the risk check sheet for dental caries according to the present invention are generally printed on papers and provided. But, it is also possible to take them in a soft ware as a computer application system and use by displaying them on a screen of the computer, or directly use or download them from the Internet. Further, while the list sheet 1 and the chart sheet 7 may be used simultaneously on paper or a screen, needless to say, they can be used individually because the check contents often include private items.

As described above in detail, the risk check sheet for dental caries according to the present invention is a risk check sheet for dental caries, which enables a technician for dental care to grasp the risk of dental caries of a patient by confirming the intra-oral state or life habit of the patient from the diagnosis results obtained through inquiries or inspections or by utilizing instruments, to comprehensively show such risk to the patient in a way that the patient per se can easily understand the cause and risk of dental caries and to give the patient a motivation for the maintenance and continuation of oral care. Accordingly, the present invention is greatly valuable in contributing to the dental remedy.

While the invention has been described in detail and with reference to specific embodiments thereof, it will be apparent to one skilled in the art that various changes and modifications can be made therein without departing from the scope thereof as defined in the appended claims.

## Claims

1. A risk check sheet for dental caries that is used for judging the risk of dental caries, which comprises:
a list sheet (1) provided with three kinds of risk items (2) consisting of a risk of dental caries caused by a resistance, a risk of dental caries caused by intra-oral bacteria, and a risk of dental caries caused by eating habit, each having a plurality of check items (3), as to each of which one from a plurality of selection items having different ranks is to be alternatively selected, and numerical data of the risks of dental caries by summing up evaluation scores given in the selection items for each risk item; **characterized by**
a chart sheet (7) provided with a standard circle (8) having a predetermined radius and centering around an origin, standard lines (9) consisting of three lines radially provided with a distance of 120 degree, centering around the origin, each starting at least from the standard circle, and allocated for the three kinds of risks of dental caries, and indicator circles (13), each of which is drawn such that, when a point of intersection (10) between each standard line and the standard circle is defined as a front point of intersection, whereas a position symmetrical to the front point of intersection on the standard circle is defined as a rear point of intersection (11), a length between each of points obtained by dividing equally a length from the front point of intersection to a maximum point (12) positioned in a predetermined distance longer than the radius of the standard circle on the standard line by a maximum value of the sum of evaluation scores in each corresponding risk item and the rear point of intersection is a diameter.

2. The risk check sheet for dental caries according to claim 1, wherein:
the check item with respect to the risk of dental caries caused by a resistance is constituted of at least two kinds selected from the group consisting of the number of decayed teeth at present, the number of filled teeth in the past, the number of missing teeth, the status of a set of teeth, the status of gingiva, the amount of saliva, the buffer capacity of saliva, the age, the presence and kind of systemic disease, the presence of pregnancy, and the presence and kind of a drug administered;
the check item with respect to the risk of dental caries caused by intra-oral bacteria is constituted of at least two kinds selected from the group consisting of the amount of dental plaque, the level of *Mutans streptococci*, the level of *Sorbrinus*, and the level of Lactobacilli; and
the check item with respect to the risk of dental caries caused by eating habit is constituted of at least two kinds selected from the group consisting of the number of eating and drinking per day, the frequency of eating between meals per day, and the contents of eating between meals.

## Patentansprüche

1. Gefahrenüberprüfungsblatt bzw. Risikoüberprüfungsblatt für Zahnkaries, welches verwendet wird, um das Risiko von Zahnkaries abzuschätzen, welches umfaßt:
ein Listenblatt (1), das mit drei Arten von Risikomerkmalen bzw. -gegenständen (2) versehen ist, bestehend aus einem Risiko von Zahnkaries, welche durch einen Widerstand bzw. eine Widerstandsfähigkeit verursacht ist, einem Risiko von Zahnkaries, welche durch intra-orale Bakterien verursacht ist, und einem Risiko von Zahnkaries, welche durch eine Eßgewohnheit verursacht ist, die jeweils eine Mehrzahl von Überprüfungsmerkmalen (3), wobei jeweils eines davon aus einer Mehrzahl von Auswahlmerkmalen, welche unterschiedliche Rangordnungen aufweisen, alternativ auszuwählen ist, und numerische Daten der Risiken von Zahnkaries durch Aufsummieren von Auswertungstreffern aufweisen, die in den Auswahlmerkmalen für jedes Risikomerkmal gegeben sind, **gekennzeichnet durch** ein Darstellungsblatt (7), das mit einem Standardkreis (8), welcher einen vorbestimmten Radius aufweist und um einen Ursprung zentriert ist, Standardlinien (9), bestehend aus drei Linien, die radial mit einem Abstand von 120 Grad vorgesehen sind, die um den Ursprung zentriert sind, wobei jede wenigstens von dem Standardkreis startet und für die drei Arten von Risiken von Zahnkaries zugewiesen ist, und Anzeige- bzw. Hinweiskreisen (13) versehen ist, von welchen jeder so gezeichnet ist, daß, wenn ein Schnittpunkt (10) zwischen jeder Standardlinie und dem Standardkreis als ein vorderer Schnittpunkt definiert ist, während eine Position symmetrisch zu dem vorderen Schnittpunkt auf dem Standardkreis als ein rückwärtiger Schnittpunkt (11) definiert ist, eine Länge zwischen jedem der Punkte, die **durch** ein gleiches Dividieren einer Länge von dem vorderen Schnittpunkt zu einem maximalen Punkt (12) erhalten sind, der in einem vorbestimmten Abstand länger als der Radius des Standardkreises auf der Standardlinie um einen maximalen Wert der Summe von Auswertungstreffern in jedem entsprechenden Risikomerkmal positioniert ist, und dem rückwärtigen Schnittpunkt ein Durchmesser ist.

2. Gefahrenüberprüfungsblatt für Zahnkaries gemäß Anspruch 1, wobei:
das Überprüfungsmerkmal in bezug auf das Risiko von Zahnkaries, die durch einen Widerstand bzw. eine Widerstandsfähigkeit verursacht ist, aus wenigstens zwei Arten besteht, die aus der Gruppe gewählt sind, bestehend aus der Anzahl von gegenwärtig schlechten Zähnen, der Anzahl von in der Vergangenheit gefüllten Zähnen, der Anzahl von fehlenden Zähnen, dem Status eines Satzes von Zähnen, dem Status der Schleimhaut, der Speichelmenge, der Pufferkapazität des Speichels, dem Alter, dem Vorhandensein und der Art einer systemischen Erkrankung, dem Vorliegen einer Schwangerschaft und dem Vorliegen und der Art eines verabreichten Medikaments bzw. Wirkstoffs;
das Überprüfungsmerkmal in bezug auf das Risiko von Zahnkaries, die durch intra-orale Bakterien bewirkt ist, aus wenigstens zwei Arten besteht, die aus der Gruppe gewählt sind, bestehend aus der Menge an Zahnbelag bzw. Plaque, dem Niveau von Mutans streptococci, dem Niveau von Sorbrinus und dem Niveau von Lactobacilli; und
das Überprüfungsmerkmal in bezug auf das Risiko von Zahnkaries, die durch eine Eßgewohnheit bewirkt ist, aus wenigstens zwei Arten besteht, die aus der Gruppe gewählt sind, bestehend aus der Zahl eines Essens und Trinkens pro Tag, der Frequenz eines Essens zwischen den Mahlzeiten pro Tag und den Gehalten eines Essens zwischen den Mahlzeiten.

## Revendications

1. Fiche de vérification des risques de carie dentaire utilisée pour juger le risque de carie dentaire, qui comprend :
une fiche d'énumération (1) comprenant trois types d'items de risque (2) consistant en un risque de carie dentaire causée par une résistance, un risque de carie dentaire causée par une bactérie intra-orale, et un risque de carie dentaire causée par une habitude alimentaire, chaque item ayant une pluralité d'items de vérification (3), un item parmi une pluralité d'items de sélection ayant des rangs différents devant être choisi alternativement pour chaque item de vérification, et des données numériques des risques de carie dentaire par addition des scores d'évaluation donnés dans les items de sélection pour chaque item de risque,
**caractérisée par**
une fiche graphique (7) comportant un cercle standard (6) ayant un rayon prédéterminé et se centrant autour d'une origine, des lignes standard (9) consistant en trois lignes d'une distance radiale de 120 degrés, se centrant autour de l'origine, chacune partant au moins du cercle standard, et attribuées pour les trois types de carie dentaire, et des cercles indicateurs (13), chacun étant dessiné de telle sorte que, lorsqu'un point d'intersection (10) entre chaque ligne standard et le cercle standard est défini comme point d'intersection avant, tandis qu'une position symétrique par rapport au point d'intersection avant sur le cercle standard est définie comme un point d'intersection arrière (11), une longueur entre chacun des points obtenus par division égale d'une longueur depuis le point d'intersection avant jusqu'à un point maximal (12) positionné à une distance prédéterminée supérieure au rayon du cercle standard sur la ligne standard par une valeur maximale de la somme des scores d'évaluation dans chaque item de risque correspondant et le point d'intersection arrière est un diamètre.

2. Fiche de vérification des risques de carie dentaire selon la revendication 1, dans laquelle :
l'item de vérification concernant le risque de carie dentaire causée par une résistance est constitué d'au moins deux types choisis dans le groupe comprenant le nombre de dents déjà cariées, le nombre de dents plombées par le passé, le nombre de dents manquantes, l'état de l'ensemble des dents, l'état des gencives, la quantité de salive, la capacité de tampon de la salive, l'âge, la présence d'une maladie systémique et son type, la présence d'une grossesse, et la présence d'un médicament administré et son type,
l'item de vérification concernant le risque de carie dentaire causée par une bactérie intra-orale est constitué d'au moins deux types choisis dans le groupe comprenant la quantité de plaque dentaire, le niveau de *Mutans streptococci,* le niveau de *Sorbrinus,* et le niveau de *Lactobacilli ;* et
l'item de vérification concernant le risque de carie dentaire causée par une habitude alimentaire est constitué d'au moins deux types choisis dans le groupe comprenant le nombre de consommations de boisson ou d'aliment par jour, la fréquence de la consommation d'aliments entre les repas par jour et la teneur des aliments consommés entre les repas.
